# EUROPÄISCHE PATENTSCHRIFT

(11) **EP 1 097 141 B1**
(45) Veröffentlichungstag und Bekanntmachung des Hinweises auf die Patenterteilung: **23.05.2007**
(21) Anmeldenummer: 99938222.9
(22) Anmeldetag: 10.07.1999
(51) Int. Cl.: C07D 233/70, C07D 233/88, A61K 31/4164

(54) **IMIDAZOLDERIVATE MIT BIPHENYLSULFONYLSUBSTITUTION, VERFAHREN ZU IHRER HERSTELLUNG, IHRE VERWENDUNG ALS MEDIKAMENT ODER DIAGNOSTIKUM**
IMIDAZOLE DERIVATIVES WITH BIPHENYLSULFONYL SUBSTITUTION, METHOD FOR PREPARING THEM AND THEIR USE AS A DRUG OR DIAGNOSTIC AGENT
DERIVES D'IMIDAZOLE A SUBSTITUTION BIPHENYLSULFONYLE, LEUR PROCEDE DE PREPARATION ET LEUR UTILISATION COMME MEDICAMENT OU AGENT DIAGNOSTIQUE

(30) Priorität: 18.07.1998 DE 19832428
(43) Veröffentlichungstag der Anmeldung: 09.05.2001
(73) Patentinhaber: Sanofi-Aventis Deutschland GmbH, 65929 Frankfurt am Main (DE)
(72) Erfinder: KLEEMANN, Heinz-Werner, D-65474 Bischofsheim (DE); LANG, Hans, Jochen, D-65719 Hofheim (DE); SCHWARK, Jan-Robert, D-65779 Kelkheim (DE); PETRY, Stefan, D-65929 Frankfurt (DE); WEICHERT, Andreas, D-63329 Egelsbach (DE)
(86) Internationale Anmeldenummer: PCT/EP1999/004887
(87) Internationale Veröffentlichungsnummer: WO 2000/003996

(56) Entgegenhaltungen:
- EP-A- 0 648 763
- EP-A- 0 855 392
- WO-A-95/23791
- WO-A-95/23792
- US-A- 5 391 732

## Beschreibung

Die Erfindung betrifft Verbindungen der Formel I, 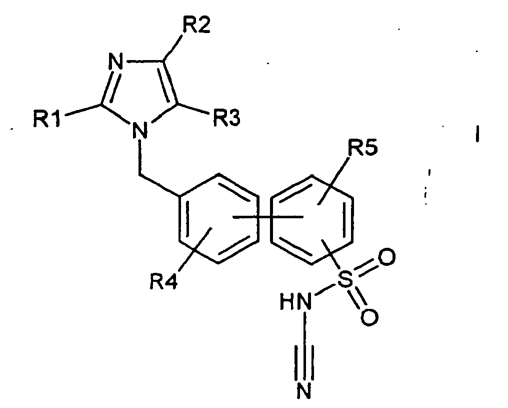 in welcher die Symbole folgende Bedeutung haben:
- R(1): Alkyl mit 1, 2, 3, 4, 5, 6, 7 oder 8 C-Atomen, -C_{d}H_{2d}-Cycloalkyl mit 3, 4, 5, 6 oder 7 C-Atomen mit d gleich Null, 1 oder 2 oder -CₐH₂ₐ-Phenyl,
wobei der Phenylteil unsubstituiert oder substituiert ist mit 1, 2 oder 3 gleichen oder verschiedenen Resten aus der Reihe F, Cl, Br, I, CF₃, Methyl, Methoxy, Hydroxy oder NR(8)R(9);
R(8) und R(9) unabhängig voneinander Wasserstoff oder Alkyl mit 1, 2, 3 oder 4 C-Atomen;
a Null, 1 oder 2;
- R(2): O-(Alkylen mit 2, 3, oder 4 C-Atomen)-O-R(17) oder NR(50)R(51); R(17) Wasserstoff oder Alkyl mit 1, 2, 3, 4, 5, 6, 7 oder 8 C-Atomen;
R(50) und R(51) unabhängig voneinander -(Alkylen mit 2, 3, oder 4 C-Atomen)-O-R(52);
R(52) Wasserstoff oder Alkyl mit 1, 2, 3, 4, 5, 6, 7 oder 8 C-Atomen;
- R(3): Wasserstoff, -CN, oder CO-R(6);
R(6) Wasserstoff, Alkyl mit 1, 2, 3, 4, 5, 6, 7 oder 8 C-Atomen oder OR(30);
R(30) Wasserstoff oder Alkyl mit 1, 2, 3, 4, 5, 6, 7 oder 8 C-Atomen;
- R(4): Wasserstoff, Cl, F, Br, I, SOₚ-R(16) oder O-CH₂-CH₂-O-R(33);
p Null, 1 oder 2;
R(16) Alkyl mit 1, 2, 3, 4, 5, 6, 7 oder 8 C-Atomen oder Phenyl, das unsubstituiert oder substituiert ist mit 1, 2 oder 3 gleichen oder verschiedenen Resten aus der Reihe F, Cl, Br, I, CF₃, Methyl, Methoxy, Hydroxy oder NR(26)R(27);
R(26) und R(27) unabhängig voneinander Wasserstoff oder Alkyl mit 1, 2, 3 oder 4 C-Atomen;
R(33) Methyl oder Ethyl;
- R(5): Wasserstoff;
sowie deren physiologisch verträglichen Salze.

Bevorzugt sind auch Verbindungen, in denen bedeutet:
- R(1): -CₐH₂ₐ-Phenyl, wobei der Phenylteil unsubstituiert oder substituiert ist mit 1 oder 2 gleichen oder verschiedenen Resten aus der Reihe F, Cl, Br, CF₃, Methyl, Methoxy, Hydroxy oder NR(8)R(9);
R(8) und R(9) unabhängig voneinander Wasserstoff oder Methyl;
a Null oder 1;
- R(2): O-(Alkylen mit 2, 3, oder 4 C-Atomen)-O-R(17) oder NR(50)R(51);
R(17) Alkyl mit 1, 2, 3 oder 4 C-Atomen, insbesondere Methyl;
R(50) und R(51) unabhängig voneinander -(Alkylen mit 2, 3, oder 4 C-Atomen)-O-R(52);
R(52) Alkyl mit 1, 2, 3, oder 4 C-Atomen, insbesondere Methyl;
- R(3): CO-R(6);
R(6) Wasserstoff;
- R(4): SO₂R(16) mit R(16) gleich Alkyl mit 1, 2, 3 oder 4 C-Atomen, insbesondere Methyl oder O-CH₂-CH₂-O-CH₃;
- R(5): Wasserstoff;
sowie deren physiologisch verträglichen Salze.

Bevorzugt sind insbesondere Verbindungen der Formel I, in denen R(2) O-CH₂-CH₂-O-R(17) oder NR(50)R(51) bedeutet mit R(17) gleich Alkyl mit 1, 2, 3 oder 4 C-Atomen, insbesondere Methyl und R(50) und R(51) gleich -CH₂-CH₂-O-R(52) mit R(52) gleich Alkyl mit 1, 2, 3 oder 4 C-Atomen, insbesondere Methyl; und R(1), R(3), R(4) und R(5) wie oben definiert sind, sowie deren physiologisch verträglichen Salze.

Bevorzugt sind desweiteren Verbindungen der Formel I, in denen die Reste R(1), R(2), R(3), R(4) und R(5) wie oben definiert sind und der Biphenylsubstituent wie in Formel Ia, Ib, Ic, Id, Ie, If, Ig, Ih oder Ii, bevorzugt wie in Formel Ii verknüpft ist, 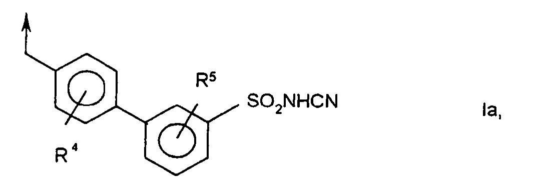 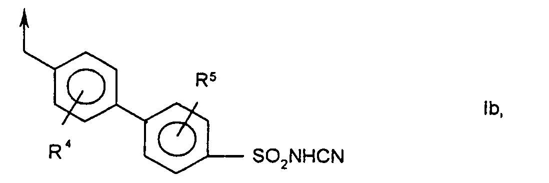 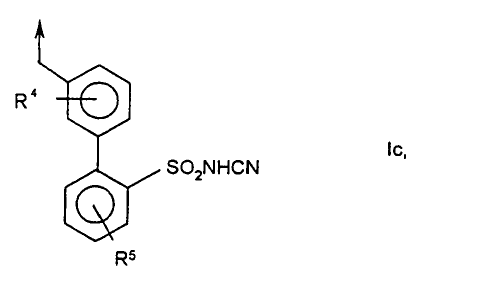 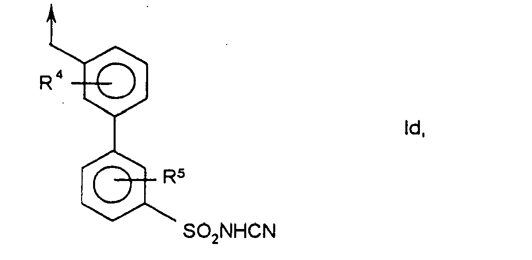 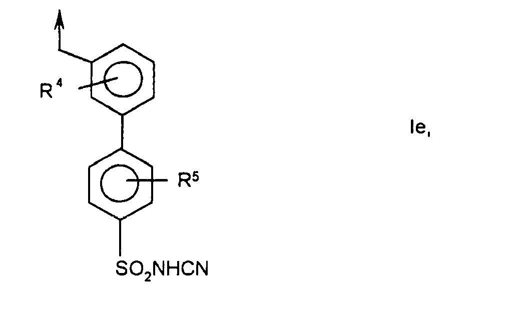 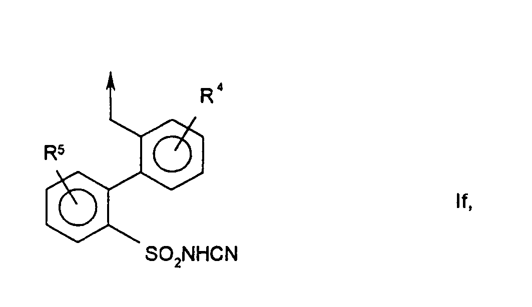 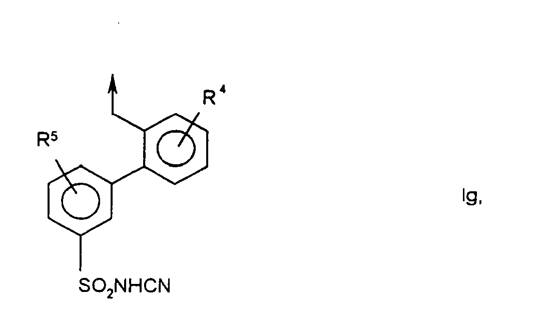 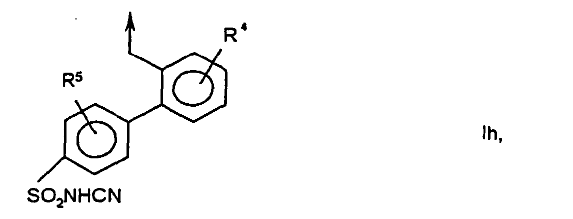 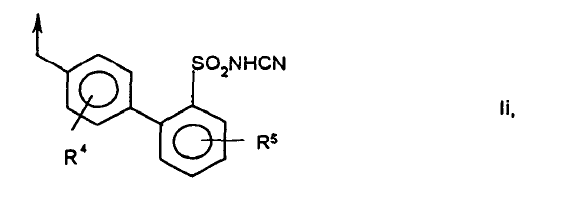 sowie deren physiologisch verträglichen Salze.

Bevorzugt sind desweiteren Verbindungen der Formel I, in denen die Reste R(1), R(2), R(3), R(4) und R(5) wie oben definiert sind und die Reste R(4) und R(5) wie in Formel Ij mit dem Biphenylsubstituent verknüpft sind, 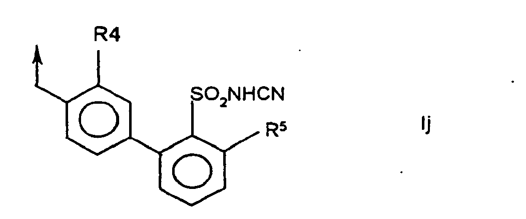 sowie deren physiologisch verträglichen Salze.

Alkylreste und Alkylenreste können geradkettig oder verzweigt sein. Dies gilt auch für die Alkylenreste der Formeln CₐH₂ₐ, und C_{d}H_{2d}. Alkylreste und Alkylenreste können auch geradkettig oder verzweigt sein, wenn sie substituiert sind oder in anderen Resten enthalten sind, z. B. in einem Alkoxyrest oder in einem Alkylmercaptorest oder in einem fluorierten Alkylrest.

Unter Cycloalkyl werden auch Alkyl-substituierte Ringe verstanden.

Beipiele für Alkylreste mit 1, 2, 3, 4, 5, 6, 7 oder 8 C-Atomen sind: Methyl, Ethyl, n-Propyl, n-Butyl, Pentyl, Hexyl, Heptyl, Octyl, Isopropyl, Isobutyl, Isopentyl, Neopentyl, Isohexyl, 3-Methylpentyl, sec-Butyl, tert-Butyl, tert-Pentyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,4-Butylen, 1,5-Pentylen, 2,2-Dimethyl-1,3-propylen, 1,6-Hexylen, usw. sind Beispiele für Alkylenreste.

Cycloalkylreste mit 3, 4, 5, 6 oder 7 C-Atomen sind insbesondere Cyclopropyl, Cyclobutyl, Cyclopentyl, Cyclohexyl, Cycloheptyl, die aber auch beispiesweise durch Alkyl mit 1, 2, 3 oder 4 C-Atomen substituiert sein können. Als Beispiel für substituierte Cycloalkylreste seien 4-Methylcyclohexyl und 2,3-Dimethylcyclopentyl genannt.

Monosubstituierte Phenylreste können in der 2-, der 3- oder der 4-Position substituiert sein, disubstituierte in der 2,3-, 2,4-, 2,5-, 2,6-, 3,4- oder 3,5-Position, trisubstituierte in der 2,3,4-, 2,3,5-, 2,3,6- 2,4,5-, 2,4,6- oder 3,4,5-Position.
Entsprechendes gilt sinngemäß analog auch für die N-haltigen Heterocylen oder den Thiophenrest.
Bei Di- oder Trisubstitution eines Restes können die Substituenten gleich oder verschieden sein.

Enthalten die Verbindungen der Formel I eine oder mehrere saure oder basische Gruppen bzw. einen oder mehrere basische Heterocyclen, so sind auch die entsprechenden physiologisch oder toxikologisch verträglichen Salze Gegenstand der Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw.

Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.
Unter physiologisch verträglichen Salzen von Verbindungen der Formel (I) sind beispielsweise auch organische und anorganische Salze zu verstehen, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. sind also in enantiomerenreiner Form, sowohl als links- als auch als rechtsdrehende Antipoden, und auch in Form von Mischungen der beiden Enantiomeren in unterschiedlichen Verhältnissen oder in Form von Racematen Gegenstand der Erfindung. Bei Vorliegen einer cis/trans-Isomerie sind sowohl die cis-Form als auch die trans-Form und Gemische dieser Formen Gegenstand der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Erfindung betrifft auch ein Verfahren zur Herstellung der Verbindungen der Formel I, sowie deren physiologisch verträglicher Salze, das dadurch gekennzeichnet ist, daß man eine Verbindung der Formel II 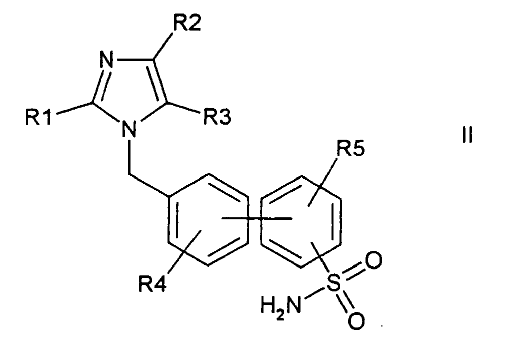 worin die Reste wie oben definiert sind und die analog zu J. Med. Chem. 1995, 38, in an sich bekannter Weise hergestellt werden können, mit Bromcyan umsetzt. Die Reaktion erfolgt vorteilhaft in einem dipolar aprotischen Lösungsmittel, das gegen Bromcyan stabil ist, zum Beispiel Acetonitril, DMA, TMU oder NMP mit einer starken Hilfsbase, die wenig nucleophil ist wie zum Beispiel K₂CO₃ oder Cs₂CO₃. Als Reaktionstemperatur kommt eine Temperatur von 0°C bis zu dem Siedepunkt des verwendeten Lösungsmittels in Frage, bevorzugt ist eine Temperatur von 60°C bis 120°C.

Die Einführung der Substituenten R(2) oder R(3) gleich Alkoxyalkoxy gelingt durch nucleophile aromatische Substitution an Verbindungen der Formel II mit R(2) gleich einer nucleofugen Abgangsgruppe, bevorzugt F, Cl oder Br und R(3) gleich Formyl oder an Verbindungen der Formel II mit R(3) gleich einer nucleofugen Abgangsgruppe, bevorzugt F, Cl oder Br und R(2) gleich Formyl. Als Nucleophil wird vorteilhaft ein Alkalisalz des betreffenden Alkohols, bevorzugt ist das Natrium-, Kalium- oder Cäsiumsalz, in dem betreffenden Alkohol als Lösungsmittel eingesetzt. Reaktionstemperatur ist eine Temperatur zwischen 0 °C und dem Siedepunkt des Alkohols, bevorzugt ist eine Temperatur zwischen 40 °C und dem Siedepunkt des Alkohols.

Die Einführung der Substituenten R(2) oder R(3) gleich Bis(alkoxyalyl)amino gelingt durch nucleophile aromatische Substitution an Verbindungen der Formel II mit R(2) gleich einer nucleofugen Abgangsgruppe, bevorzugt F, Cl oder Br und R(3) gleich Formyl oder an Verbindungen der Formel II mit R(3) gleich einer nucleofugen Abgangsgruppe, bevorzugt F, Cl oder Br und R(2) gleich Formyl. Als Nucleophil wird entweder das betreffende Bis(alkoxyalyl)amin selbst oder ein Alkalisalz des betreffenden Amins, bevorzugt ist das Lithium- Natrium-, Kalium- oder Cäsiumsalz, in einem dipolar aprotischen Lösungsmittel eingesetzt. Als Lösungsmittel kommen daher infrage THF, DMF, Tetramethylharnstoff, N-Methylpyrrolidon und Hexamethylphosphorsäuretriamid. Reaktionstemperatur ist eine Temperatur zwischen 0 °C und dem Siedepunkt des Lösungsmittels, bevorzugt ist eine Temperatur zwischen 40 °C und dem Siedepunkt des Lösungsmittels.

Die Einführung des Substituenten R(4) erfolgt vorteilhaft auf der Stufe des Toluolderivats III, 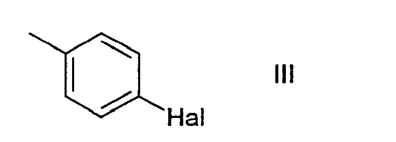 wobei Hal eine mit der Suzuki-Reaktion vereinbare Abgangsgruppe, bevorzugt Brom oder lod bedeutet. Die Einführung eines SO₂-Alkyl-Restes über Chlorsulfonierung ist beispielhaft in J. Med. Chem. 1997, 40, 2017 oder J. Org. Chem. (1991), 56(16), 4974-6 beschrieben.

Alle Reaktionen zur Synthese der Verbindungen der Formel I sind dem Fachmann an sich wohlbekannt und können unter Standardbedingungen nach oder analog zu Literaturvorschriften durchgeführt werden, wie sie zum Beispiel in Houben-Weyl, Methoden der organischen Chemie, Thieme-Verlag, Stuttgart, oder Organic Reactions, John Wiley & Sons, New York, beschrieben sind. Je nach den Gegebenheiten des Einzelfalls kann es bei der Synthese der Verbindungen der Formel I zur Vermeidung von Nebenreaktionen auch vorteilhaft oder notwendig sein, bestimmte funktionelle Gruppen vorübergehend durch die Enführung von Schutzgruppen zu blockierenn und später dann wieder freizusetzen oer funktionelle Gruppen zunächst in Form von Vorstufen einzusetzen, aus denen in einem späteren Schritt dann die gewünschte funktionelle Gruppe erzeugt wird. Solche Synthesestrategien und die für den Einzelfall geeigneten Schutzgruppen oder Vorstufen sind dem Fachmann bekannt. Die erhaltenen Verbindungen der Formel I können gegebenenfalls nach üblichen Reinigungsmethoden, zum Beispiel durch Umkristallisation oder Chromatographie, gereinigt werden.Die Ausgangsverbindungen für die Herstellung der Verbindungen der Formel I sind käuflich erhältlich oder können nach oder analog zu Literaturvorschriften hergestellt werden.

Des weiteren betrifft die Erfindung die Verwendung einer Verbindung der Formel I und/oder eines physiologisch verträglichen Salzes davon zur Herstellung eines Medikaments zur Behandlung oder Prophylaxe von durch ischämische Zustände hervorgerufenen Krankheiten;
sowie die Verwendung einer Verbindung der Formel I und/oder eines physiologisch verträglichen Salzes davon zur Herstellung eines Medikaments zur Behandlung oder Prophylaxe des Herzinfarkts;
sowie die Verwendung einer Verbindung der Formel I und/oder eines physiologisch verträglichen Salzes davon zur Herstellung eines Medikaments zur Behandlung oder Prophylaxe der Angina Pectoris;
sowie die Verwendung einer Verbindung der Formel I und/oder eines physiologisch verträglichen Salzes davon zur Herstellung eines Medikaments zur Behandlung oder Prophylaxe von ischämischen Zuständen des Herzens;
sowie die Verwendung einer Verbindung der Formel I und/oder eines physiologisch verträglichen Salzes davon zur Herstellung eines Medikaments zur Behandlung oder Prophylaxe von ischämischen Zuständen des peripheren und zentralen Nervensystems und des Schlaganfalls;
sowie die Verwendung einer Verbindung der Formel I und/oder eines physiologisch verträglichen Salzes davon zur Herstellung eines Medikaments zur Behandlung oder Prophylaxe von ischämischen Zuständen peripherer Organe und Gliedmaßen;
sowie die Verwendung einer Verbindung der Formel I und/oder eines physiologisch verträglichen Salzes davon zur Herstellung eines Medikaments zur Behandlung von Schockzuständen;
sowie die Verwendung einer Verbindung der Formel I und/oder eines physiologisch verträglichen Salzes davon zur Herstellung eines Medikaments zum Einsatz bei chirurgischen Operationen und Organtransplantationen;
sowie die Verwendung einer Verbindung der Formel I und/oder eines physiologisch verträglichen Salzes davon zur Herstellung eines Medikaments zur Konservierung und Lagerung von Transplantaten für chirurgische Maßnahmen;
sowie die Verwendung einer Verbindung der Formel I und/oder eines physiologisch verträglichen Salzes davon zur Herstellung eines Medikaments zur Behandlung von Krankheiten, bei denen die Zellproliferation eine primäre oder sekundäre Ursache darstellt; und somit ihre Verwendung zur Herstellung eines Antiatherosklerotikums, eines Mittels gegen diabetische Spätkomplikationen, Krebserkrankungen, fibrotische Erkrankungen wie Lungenfibrose, Leberfibrose oder Nierenfibrose, Prostatahyperplasie;
sowie die Verwendung einer Verbindung der Formel I und/oder eines physiologisch verträglichen Salzes davon zur Herstellung eines Medikaments zur Behandlung von gestörtem Atemantrieb;
sowie eine pharmazeutische Zubereitung, gekennzeichnet durch einen wirksamen Gehalt einer Verbindung der Formel I und/oder eines physiologisch verträglichen Salzes davon.

Die erfindungsgemäßen Verbindungen der Formel I eignen sich als Inhibitoren des Natrium-abhängigen Bicarbonat/Chlorid-Austauschers (NCBE) bzw. des Natrium/Bicarbonat-Symporters.

Den erfindungsgemäßen Verbindungen der Formel I ähnliche Verbindungen sind aus den US-Patentschriften 5,482,957 und 5,604,251 bekannt. Sie weisen jedoch nicht die nach der Erfindung stets vorhandene Sulfonylcyanamid-Seitenkette auf. Imidazolderivate als Angiotensin II-antagonisten werden auch in WO9523792, WO9523791, US 5391732, EP-A 648763 beschrieben. Die bekannten Verbindungen sind Angiotensin II-Rezeptor-Antagonisten des Subtyps AT1, welche Wirkung bei den erfindungsgemäßen Verbindungen I nicht oder nur im geringen Ausmaß vorhanden ist.

In der älteren europäischen Patentanmeldung EP-A 855392 werden Imidazolderivate mit einer Biphenylsulfonylcyanamid-Seitenkette als NCBE-Inhibitoren vorgeschlagen.
Die in der vorliegenden Erfindung beschriebenen neuen Imidazolderivate mit Biphenylsulfonylcyanamid-Seitenkette weisen einen speziellen Substituenten R(2) und/oder R(3) am Imidazolring auf und zeichnen sich durch eine hohe Wirksamkeit in der Inhibition des zellulärenen Na⁺-abhängigen Bicarbonat/Chlorid-Austauschs sowie einer verbesserten Bioverfügbarkeit aus.

Die erfindungsgemäßen Verbindungen der Formel (I) zeigen sehr gute antiarrhythmische Eigenschaften, wie sie beispielsweise für die Behandlung von Krankheiten wichtig sind, welche bei Sauerstoffmangelerscheinungen auftreten. Die Verbindungen der Formel (I) sind infolge ihrer pharmakologischen Eigenschaften als antiarrhythmische Arzneimittel mit cardioprotektiver Komponente zur Infarktprophylaxe und der Infarktbehandlung sowie zur Behandlung der Angina Pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern.
Wegen ihrer schützenden Wirkungen gegen pathologische hypoxische und ischämische Situationen können die erfindungsgemäßen Verbindungen der Formel (I) infolge Inhibition des zellulären Na⁺-abhängigen Cl⁻/HCO₃⁻-Austauschmechanismus (NCBE) bzw. des Natrium/Bicarbonat-Symporters als Arzneimittel zur Behandlung aller akuten oder chronischen durch Ischämie ausgelösten Schäden oder dadurch primär oder sekundär induzierten Krankheiten verwendet werden. Sie schützen akut oder chronisch sauerstoffmangel-versorgte Organe durch Verringerung oder Verhinderung ischämisch induzierter Schäden und eignen sich somit als Arzneimittel beispielsweise bei Thrombosen, Gefäßspasmen, Atherosklerose oder bei operativen Eingriffen (z.B. bei OrganTransplantationen von Niere und Leber, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können) oder chronischem oder akutem Nierenversagen.

Die Verbindungen der Formel (I) sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel (I) ebenfalls zur Behandlung von Formen des Schocks, wie beispielsweise des allergischen, cardiogenen, hypovolämischen und des bakteriellen Schocks.

Darüberhinaus zeichnen sich die erfindungsgemäßen Verbindungen der Formel (I) durch starke inhibierende Wirkung auf die Proliferationen von Zellen, beispielsweise der Fibroblasten- Zellproliferation und der Proliferation der glatten Gefäßmuskelzellen und der Mesangiumzellen aus. Deshalb kommen die Verbindungen der Formel (I) als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und/oder -hyperplasien, insbesondere bei Prostatahyperplasie bzw. Prostatahypertrophie verwendet werden.

Die erfindungsgemäßen Verbindungen der Formel I sowie ihre physiologisch verträglichen Salze eignen sich zur Verwendung in der Theraphie und/oder Prophylaxe des Herzinfarkts, der Angina Pectoris, von durch ischämische Zustände hervorgerufenen Krankheiten, von gestörtem Atemantrieb, von ischämischen Zuständen des Herzens, von ischämischen Zuständen des peripheren und zentralen Nervensystems und des Schlaganfalls, von ischämischen Zuständen peripherer Organe und Gliedmaßen, von Krankheiten, bei denen die Zellproliferation eine primäre oder sekundäre Ursache darstellt oder in der Behandlung von Schockzuständen, oder zum Einsatz bei chirurgischen Operationen und Organtransplantationen oder zur Konservierung und Lagerung von Transplantaten für chirurgische Maßnahmen.

Es wurde gefunden, daß Inhibitoren des Na⁺-abhängigen Cl⁻/HCO3⁻ -Austauschers (NCBE-Inhibitoren) bzw. des Natrium/Bicarbonat-Symporters die Atmung durch eine Zunahme der Chemosensibiliät der Atmungs-Chemorezeptoren stimulieren können. Diese Chemorezeptoren sind in beträchtlichem Umfang für die Aufrechterhaltung einer geordneten Atemtätigkeit verantwortlich. Sie werden durch Hypoxie, pH-Abfall und Anstieg von CO₂ (Hyperkapnie) im Körper aktiviert und führen zu einer Anpassung des Atemminutenvolumens. Im Schlaf ist die Atmung besonders störanfällig und in hohem Maße abhängig von der Aktivität der Chemorezeptoren. Eine Verbesserung des Atemantriebes durch Stimulation der Chemorezeptoren mit Substanzen, die den Na⁺-abhängigen Cl⁻/HCO₃⁻ -Austausch hemmen, führt zu einer Verbesserung der Atmung bei folgenden klinischen Zuständen und Krankheiten: Gestörter zentraler Atemantrieb (z.B. zentrale Schlafapnoen, plötzlicher Kindstod, postoperative Hypoxie), muskulär-bedingte Atemstörungen, Atemstörungen nach Langzeitbeatmung, Atemstörungen bei Adapation im Hochgebirge, obstruktive und gemischte Form der Schlafapnoen, akute und chronische Lungenkrankheiten mit Hypoxie und Hyperkapnie.

Die erfindungsgemäßen Verbindungen der Formel I und ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Herstellung von Medikamenten dafür. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen pharmazeutisch einwandfreien Träger- und Hilfsstoffen enthalten. Die pharmazeutischen Zubereitungen enthalten normalerweise 0,1 bis 99 Gewichtsprozent, bevorzugt 0,5 bis 95 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die eine Verbindung der Formel (I) und/oder ihre physiologisch verträglichen Salze enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken-als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs einer Verbindung der Formel (I) und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Die Verbindungen der Formel I und/oder ihre physiologisch verträglichen Salze können zur Erzielung einer vorteilhaften therapeutischen Wirkung auch zusammen mit anderen pharmakologisch wirksamen Verbindungen zur Behandlung oder Prophylaxe der obengenannten Krankheitsbilder, insbesonders zur Behandlung von Herz-Kreislauf-Erkrankungen eingesetzt werden. Bevorzugt ist die Kombination mit Inhibitoren des Natrium/Wasserstoff-Austauschers (NHE) und/oder mit aktiven Substanzen aus anderen Herz-Kreislauf-Wirkstoffklassen.

Die Erfindung betrifft desweiteren die Kombination von a) NCBE-Inhibitoren der Formel I und/oder deren physiologisch verträglichen Salze mit NHE-Inhibitoren und/oder deren physiologisch verträglichen Salze; b) NCBE-Inhibitoren der Formel I und/oder deren physiologisch verträglichen Salze mit aktiven Substanzen aus anderen Herz-Kreislauf-Wirkstoffklassen und/oder deren physiologisch verträglichen Salze sowie c) von NCBE-Inhibitoren der Formel I und/oder deren physiologisch verträglichen Salze mit NHE-Inhibitoren und/oder deren physiologisch verträglichen Salze und mit aktiven Substanzen aus anderen Herz-Kreislauf-Wirkstoffklassen und/oder deren physiologisch verträglichen Salze.

Bei den bekannten und als NHE-Inhibitoren identifizierten Wirkstoffen handelt es sich um Guanidin-Derivate, vorzugsweise um Acylguanidine, unter anderem wie sie in Edward J. Cragoe, Jr., DIURETICS, Chemistry, Pharmacology and Medicine, J. WILEY & Sons (1983), 303 - 341 beschrieben sind oder um die in DE19737224.4 aufgeführten NHE-Inhibitoren.

Geeignete NHE-Inhibitoren sind beispielsweise auch Benzoylguanidine, wie sie in US 5292755, US 5373024, US 5364868, US 5591754, US 5516805, US 5559153, US 5571842, US 5641792, US 5631293, EP-A 577024, EP-A 602522, EP-A 602523, EP-A 603650, EP-A 604852, EP-A 612723, EP-A 627413, EP-A 628543, EP-A 640593, EP-A 640588, EP-A702001, EP-A 713864, EP-A 723956, EP-A 754680, EP-A 765868, EP-A 774459, EP-A 794171, EP-A 814077, EP-A 869116 beschrieben sind; ortho-substituierte Benzoylguanidine, wie sie in EP-A 556673, EP-A 791577, EP-A 794172 beschrieben sind; ortho-amino-substituierte Benzoylguanidine, wie sie in EP-A 690048 beschrieben sind; Isochinoline, wie sie in EP-A 590455 beschrieben sind; Benzokondensierte 5-Ring-Heterocyclen, wie sie in EP-A 639573 beschrieben sind; Diacylsubstituierte Guanidine, wie sie in EP-A 640587 beschrieben sind; Acylguanidine, wie sie in US 5547953 beschrieben sind; Perfluoralkylgruppen tragende Phenylsubstituierte Alkyl- bzw. Alkenyl-carbonsäure-Guanidine, wie sie in US 5567734, EP-A 688766 beschrieben sind; Heteroaroyl-guanidine, wie sie in EP-A 676395 beschrieben sind; Bizyklische Heteroaroyl-guanidine, wie sie in EP-A 682017 beschrieben sind; Indenoylguanidine, wie sie in EP-A 738712 beschrieben sind; Benzyloxycarbonyl-guanidine, wie sie in EP-A 748795 beschrieben sind; Fluorphenylgruppen tragende Phenylsubstituierte Alkenylcarbonsäure-Guanidine, wie sie in EP-A 744397 beschrieben sind; substituierte Zimtsäureguanidine, wie sie in EP-A 755919 beschrieben sind; Sulfonimidamide, wie sie in EP-A 771788 beschrieben sind; Benzoldicarbonsäurediguanidine, wie sie in EP-A 774458, EP-A 774457 beschrieben sind; Diarylcarbonsäure-diguanidine, wie sie in EP-A 787717 beschrieben sind; substituierte Thiophenylalkenylcarbonsäureguanidine, wie sie in EP-A 790245 beschrieben sind; Bis-ortho-substituierte Benzoylguanidine, wie sie in EP-A 810207 beschrieben sind; Substituierte 1- oder 2-Naphthylguanidine, wie sie in EP-A 810205 und EP-A 810206 beschrieben sind; Indanylidin-acetylguanidine, wie sie in EP-A 837055 beschrieben sind; Phenylsubstituierte Alkenylcarbonsäure-guanidine, wie sie in EP-A 825178 beschrieben sind;
Aminopiperidyl-Benzoylguanidine, wie sie in EP-A 667341 beschrieben sind; Heterocycloxy-Benzylguanidine, wie sie in EP-A 694537 beschrieben sind; ortho-substituierte Benzoylguanidine, wie sie in EP-A 704431 beschrieben sind; ortho-substituierte Alkyl-Benzylguanidine, wie sie in EP-A 699660 beschrieben sind; ortho-substituierte Heterocyclyl-Benzoylguanidine, wie sie in EP-A 699666 beschrieben sind; ortho-substituierte 5-Methylsulfonyl-benzoylguanidine, wie sie EP-A 708088 beschrieben sind; ortho-substituierte 5-Alkylsulfonyl-benzoylguanidine mit 4-amino Substituenten, wie sie in EP-A 723963 beschrieben sind; ortho-substituierte 5-Alkylsulfonyl-Benzoylguanidine mit 4-Mercapto Substituenten, wie sie in EP-A 743301 beschrieben sind; 4-Sulfonyl- oder 4-Sulphinyl-Benzylguanidine, wie sie in EP-A 758644 beschrieben sind; Alkenylbenzoylguanidine, wie sie in EP-A 760365 beschrieben sind; Benzoylguanidine mit annelierten, cyclischen Sulfonen, wie sie in DE 19548708 beschrieben sind; Benzoyl-, polycyclische Aroyl- und Heteroaroyl-guanidine, wie sie in WO 9426709 beschrieben sind; 3-Aryl/Heteroaryl-Benzoylguanidine, wie sie in WO 9604241 beschrieben sind; 3-Phenyl-Benzoylguanidine mit einem basischen Amid in der 5-Position, wie sie in WO 9725310 beschrieben sind; 3-Dihalothienyl- oder 3-Dihaloptienyl-Benzoylguanidine mit einem basichen Substituenten in der 5-Position, wie sie in WO 9727183 beschrieben sind; 3-Methylsulfonylbenzoylguanidinen mit bestimmten Aminosubstituenten in der 4-Position, wie sie in WO 9512584 beschrieben sind; Amilorid-Derivate, wie sie in WO 9512592 beschrieben sind; 3-Methylsulfonyl-benzoylguanidine mit bestimmten Aminosubstituenten in der 4-Position, wie sie in WO 9726253 beschrieben sind; Indoloylguanidine, wie sie in EP-A 622356 und EP-A 708091 beschrieben sind; Indoloylguanidine mit einem annelierten zusätzlichen Ringsystem, wie sie in EP 787728 beschrieben sind; Methylguanidin-Derivate, wie sie in WO 9504052 beschrieben sind; 1,4-Benzoxazinoylguanidine, wie sie in EP-A 719766 beschrieben sind; 5-Brom-2-Naphthoylguanidine, wie sie in JP 8225513 beschrieben sind; Quinolin-4-Carbonylguanidine mit einem Phenylrest in 2-Position, wie sie in EP-A 726254 beschrieben sind; Cinnamoylguanidine, wie sie in JP 09059245 beschrieben sind; Propenoylguanidine mit einem Naphthalin-Substituenten, wie sie in JP 9067332 beschrieben sind; Propenoylguanidine mit Indolsubstituenten, wie sie in JP 9067340 beschrieben sind; oder Heteroarylsubstituierte Acroylguanidine, wie sie in WO 9711055 beschrieben sind, sowie deren physiologisch verträglichen Salze.

Bevorzugte NHE-Inhibitoren sind die in den genannten Publikationen als bevorzugt hervorgehobenen Verbindungen. Ganz besonders bevorzugt sind die Verbindungen Cariporid (HOE642), HOE 694, EMD 96785, FR 168888, FR 183998, SM-20550, KBR-9032, sowie deren physiologisch verträglichen Salze. Am meisten bevorzugt ist Cariporid oder ein anderes physiologisch verträgliches Salz des N-(4-Isopropyl-3-methansulfonyl-benzoyl)-guanidin.

Beispiele für herzkreislaufaktive Wirkstoffklassen, die sich therapeutisch vorteilhaft mit NCBE-Inhibitoren kombinieren lassen oder zusätzlich mit Kombinationen von NCBE-Inhibitoren und NHE-Inhibitoren kombiniert werden können, sind Beta-Rezeptoren-Blocker, Calcium-Antagonisten, Angiotensin-Conversions-Enzym-Hemmer, Angiotensin-Rezeptorblocker, Schleifendiuretika, Thiaziddiuretika, kaliumsparende Diuretika, Aldosteronantagonisten, wie sie beispielsweise in der Blutdrucksenkung eingesetzt werden, sowie Herzglykoside oder andere kontraktionskraftverstärkende Mittel in der Behandlung der Herzinsuffizienz und des Congestive Heart Failures, sowie Antiarrhythmika der Klassen I - IV, Nitrate, K_{ATP}-Öffner, K_{ATP}-Blocker, Hemmer des Veratridin-aktivierbaren Natriumkanals usw. So sind zum Beispiel geeignet: die Betablocker Propanolol, Atenolol, Metoprolol; die Calciumantagonisten Diltiazem-Hydrochlorid, Verapamil-Hydrochlorid, Nifedipin; die ACE-Hemmer Captopril, Enalapril, Ramipril; Trandolapril, Quinapril, Spirapril, bevorzugt Ramipril oder Trandolapril; die Angiotensin II-Rezeptorantagonisten Losartan, Valsartan, Telmisartan, Eprosartan, Tasosartan, Candesartan, Irbesartan; die Schleifendiruetika Furosemid, Piretanid, Torasemid; die Thiazid-Diuretika Hydrochlorothiazid, Metolazon, Indapamid; die kaliumsparenden Diuretika Amilorid, Triamteren, Spironolacton; die Herzglykoside Digoxin, Digitoxin, Strophanthin; die Antiarrhythmika Amiodaron, Sotalol, Bretylium, Flecainid; das Nitrat Glyceroltrinitrat; die K⁺(ATP)-Öffner Cromakalim, Lemakalim, Nocorandil, Pinacidil, Minoxidil; die Hemmer des veratridin-aktivierbaren Na⁺-Kanals.
Ein Beispiel eines solchen besonders vorteilhaften Kombinationspartners mit NCBE-Inhibitoren sind Blocker des nicht-inaktivierenden-Natriumkanals (Veratridin-aktivierbarer Natriumkanal). Die Kombinationen eines NCBE-Inhibitors mit einem Blocker des nicht-inaktivierenden-Natriumkanals (Veratridin-aktivierbarer Natriumkanal) eignen sich zur Infarkt- und Re-infarktprophylaxe und der Infarktbehandlung sowie zur Behandlung der Angina Pectoris und der Inhibierung ischämisch induzierter Herzarrhythmien, der Tachykardie und der Entstehung und Aufrechterhaltung des Kammerflimmerns, wobei die Kombinationen eines NCBE-Inhibitors mit einem Blocker des nicht-inaktivierenden-Natriumkanals auch präventiv die pathophysiologischen Vorgänge beim Entstehen ischämisch induzierter Schäden inhibieren oder stark vermindern. Wegen ihrer verstärkten schützenden Wirkungen gegen pathologische hypoxische und ischämische Situationen können die erfindungsgemäßen Kombinationen eines NCBE-Inhibitors mit einem Blocker des nicht-inaktivierenden-Natriumkanals infolge verstärkter Inhibierung des Na+ Einstroms in die Zelle als Arzneimittel zur Behandlung aller akuten oder chronischen, durch Ischämie ausgelösten Schäden oder dadurch primär oder sekundäre induzierter Krankheiten verwendet werden. Dies betrifft ihre Verwendung als Arzneimittel für operative Eingriffe, z. B. bei Organtransplantationen, wobei die Kombinationen eines NCBE-Inhibitors mit einem Blocker des nicht-inaktivierenden-Natriumkanals sowohl für den Schutz der Organe im Spender vor und während der Entnahme, zum Schutz entnommener Organe beispielweise auch bei deren Lagerung in physiologischen Badflüssigkeiten, wie auch bei der Überführung in den Empfängerorganismus verwendet werden können. Die Kombinationen eines NCBE-Inhibitors mit einem Blocker des nicht-inaktivierenden-Natriumkanals sind ebenfalls wertvolle, protektiv wirkende Arzneimittel bei der Durchführung angioplastischer operativer Eingriffe beispielweise am Herzen wie auch an peripheren Gefäßen. Entsprechend ihrer protektiven Wirkung gegen ischämisch induzierte Schäden sind die Kombinationen eines NCBE-Inhibitors mit einem Blocker des nicht-inaktivierenden-Natriumkanals auch als Arzneimittel zur Behandlung von Ischämien des Nervensystems, insbesondere des Zentralnervensystems geeignet, wobei sie zur Behandlung des Schlaganfalls oder des Hirnödems geeignet sind. Darüber hinaus eignen sich die erfindungsgemäßen Kombinationen eines NCBE-Inhibitors mit einem Blocker des nicht-inaktivierenden-Natriumkanals ebenfalls zur Behandlung von Formen des Schocks, wie beispielsweise des allergischen, cardiogenen, hypovolämischen und des bakteriellen Schocks.

Neben der Anwendung als fixe Kombination betrifft die Erfindung auch die gleichzeitige, getrennte oder zeitlich abgestufte Anwendung von NCBE-Inhibitoren der Formel I und/oder deren physiologisch verträgliches Salze mit NHE-Inhibitoren und/oder einer zusätzlichen aktiven Substanz aus einer anderen Herz-Kreislauf-Wirkstoffklasse zur Behandlung der obengenannten Krankheiten.

Die Erfindung betrifft desweiteren eine pharmazeutische Zubereitung, enthaltend a) einen NCBE-Inhibitor der Formel I und/oder deren physiologisch verträgliches Salz und einen NHE-Inhibitor und/oder deren physiologisch verträglichen Salze; oder b) einen NCBE-Inhibitor der Formel I und/oder deren physiologisch verträgliches Salz und zusätzlich eine aktive Substanz aus einer anderen Herz-Kreislauf-Wirkstoffklasse und/oder deren physiologisch verträglichen Salze; oder c) einen NCBE-Inhibitor der Formel I und/oder deren physiologisch verträgliches Salz, einen NHE-Inhibitor und zusätzlich eine aktive Substanz aus einer anderen Herz-Kreislauf-Wirkstoffklasse, und/oder deren physiologisch verträglichen Salze.

Durch die kombinierte Anwendung kann der Effekt des einen Kombinationspartners durch den jeweiligen anderen Partner potenziert werden, d.h., die Wirkung und/oder Wirkdauer einer erfindungsgemäßen Kombination oder Zubereitung ist stärker bzw. anhaltender als die Wirkung und/oder Wirkdauer der jeweiligen Einzelkomponenten (synergistischer Effekt). Dies führt bei einer kombinierten Anwendung zu einer Verringerung der Dosis der jeweiligen Kombinationspartner, verglichen mit der Einzelanwendung. Die erfindungsgemäßen Kombinationen und Zubereitungen besitzen demnach den Vorteil, daß die zu applizierenden Wirkstoffmengen signifikant reduziert und unerwünschte Nebenwirkungen eliminiert bzw. stark redzuziert werden können.

Die Erfindung betrifft ferner eine Handelspackung, enthaltend als pharmazeutischen Wirkstoff a) einen NCBE-Inhibitor der Formel I und einen NHE-Inhibitor und/oder deren physiologisch verträglichen Salze; oder b) einen NCBE-Inhibitor der Formel I und zusätzlich eine aktive Substanz aus einer anderen Herz-Kreislauf-Wirkstoffklasse und/oder deren physiologisch verträglichen Salze; oder c) einen NCBE-Inhibitor der Formel I, einen NHE-Inhibitor und zusätzlich eine aktive Substanz aus einer anderen Herz-Kreislauf-Wirkstoffklasse und/oder deren physiologisch verträglichen Salze jeweils zusammen mit Instruktionen für die Verwendung dieser Wirkstoffe in Kombination zur gleichzeitigen, getrennten oder zeitlich abgestuften Anwendung in der Behandlung oder Prophylaxe der obengenannten Krankheitsbilder, insbesondere zur Behandlung von Herz-Kreislauf-Erkrankungen.

Die erfindungsgemäßen pharmazeutischen Zubereitungen können beispielsweise hergestellt werden, indem man entweder die Einzelkomponenten als Pulver intensiv mischt, oder indem man die Einzelkomponenten in den geeigneten Lösungsmittel wie beispielsweise einem niederen Alkohol auflöst und das Lösungsmittel anschließend entfernt.

Das Gewichstverhältnis vom NBCE-Inhibitor zum NHE-Inhibitor oder der herzkreislaufaktiven Substanz in den erfindungsgemäßen Kombinationen und Zubereitungen beträgt zweckmässigerweise 1:0,01 bis 1:100, vorzugsweise 1:0,1 bis 1:10.

Die erfindungsgemäßen Kombination und Zubereitungen enthalten insgesamt vorzugsweise 0,5-99,5 Gew.-%, insbesondere 4-99 Gew.-% dieser Wirkstoffe.

Bei der erfindungsgemäßen Anwendung bei Säugern, vorzugsweise beim Menschen bewegen sich beispielsweise die Dosen der verschieden Wirkstoffkomponenten im Berreich von 0,001 bis 100 mg/kg/Tag.

### Liste der Abkürzungen:

- BCECF: 2',7'-Bis(2-carboxyethyl)-5,6-carboxyfluorescein
- CH₂Cl₂: Dichlormethan
- DCI: Desorption-Chemicallonisation
- DMF: N,N-Dimethylformamid
- EE: Ethylacetat (EtOAc)
- EI: electron impact
- ES: Elektrospray-lonisation
- HEP: n-Heptan
- MeOH: Methanol
- mp: Schmelzpunkt
- NCBE: Natriumabhängiger Chlorid/Bicarbonat-Austauscher
- NHE: Natrium/Wasserstoff-Austauscher
- RT: Raumtemperatur
- ZNS: Zentralnervensystem

### Allgemeine Vorschrift zur Herstellung von Sulfonylcyanamiden aus Sulfonamiden

Das Sulfonamid-Ausgangsmaterial wird in 10 ml/mmol wasserfreiem Acetonitril gelöst, 3 Mol-Äquivalente K₂CO₃ sowie ein Mol-Äquivalent einer 5 N Lösung von BrCN in Acetonitril zugetropft und bis zum vollständigen Umsatz unter Rückfluß erhitzt (typische Reaktionszeit 10 Minuten bis 6 Stunden). Das Reaktionsgemisch wird anschließend ohne weitere Aufarbeitung an Kieselgel chromatographiert.

### Beispiele

### Beispiel 1:

4'-[5-Formyl-4-(2-methoxy-ethoxy)-2-phenyl-imidazol-1-ylmethyl]-3'-methansulfonylbiphenyl-2-sulfonylcyanamid 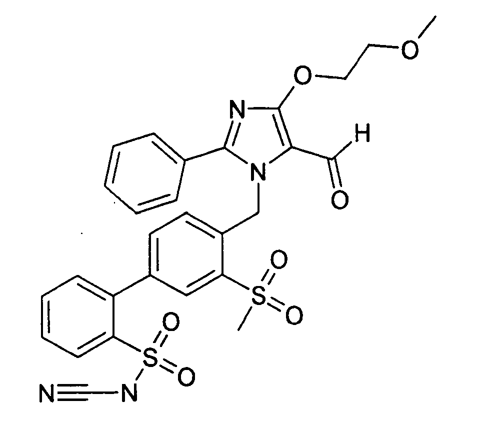
a) 2-Brom-5-methyl-benzolsulfonyl chlorid
40 g 4-Bromtoluol werden unter Rühren langsam bei -10 °C in 250 ml Chlorsulfonsäure eingetragen. 30 Minuten wird bei dieser Temperatur nachgerührt, man läßt auf 0 °C erwärmen und gießt auf überschüssiges Eis. Das Produkt wird abgesaugt und mit wenig Wasser gewaschen. Über P₄O₁₀ wird im Vakuum getrocknet und man erhält 63 g eines farblosen Feststoffs, der direkt weiter umgesetzt wird.
b) 2-Brom-5-methyl-benzolsulfinsäure
37.6 g Natriumsulfit werden in 500 ml Wasser gelöst und auf 70 °C erwärmt. Bei dieser Temperatur werden portionsweise 62 g 2-Brom-5-methyl-benzolsulfonyl chlorid zugegeben. Hierbei wird gleichzeitig eine 10 N wäßrige NaOH-Lösung zugetropft, sodaß der pH-Wert der Lösung zwischen pH=9 und pH=10 gehalten wird. 1.5 Stunden wird bei 70 °C nachgerührt, die Lösung abfiltriert und anschließend im Eisbad mit einer gesättigten wäßrigen HCI-Lösung auf pH=0 gestellt. 30 Minuten wird nachgerührt, anschließend das Produkt abfiltriert, mit wenig Wasser nachgewaschen und getrocknet. Man erhält 49.6 g weißer Kristalle,

| | |
|---|---|
| mp 120-122 °C. | MS (ES) : 236 (M+H)⁺ |

c) Natrium-2-brom-5-methyl-benzolsulfinat
49.6 g 2-Brom-5-methyl-benzolsulfinsäure werden in 400 ml Methanol gelöst und mit einer äquimolaren Menge NaOH in 50 ml Wasser versetzt. 3 Stunden wird bei RT nachgerührt, die Lösung abfiltriert und anschließend die Solventien im Vakuum entfernt. Zuletzt werden Wasserreste azeotrop mit 50 ml Toluol entfernt. Der feste Rückstand wird über P₄O₁₀ wird im Vakuum getrocknet und man erhält 54.0 g Produkt, mp 288-290 °C (unter Zersetzung).
d) 1-Brom-2-methansulfonyl-4-methyl-benzol
54.0 g Natrium-2-brom-5-methyl-benzolsulfinat werden in 300 ml wasserfreiem DMF suspendiert und mit 45.7 ml Methyliodid versetzt. Dabei steigt die Temperatur der Lösung auf 50 °C an. 3 Stunden wird bei 50 °C nachgerührt und das DMF im Vakuum entfernt. Der Rückstand wird mit 500 ml Wasser verrührt, 1 Stunde bei 0 °C nachgerührt und abfiltriert. Das Produkt wird mit Wasser gewaschen, getrocknet und aus 400 ml HEP/250 ml EE mit Aktivkohle umkristallisiert. Man erhält 27.0 g farbloser Kristalle, mp 110-114 °C.

| | |
|---|---|
| R_{f}(EE/HEP 1:4) = 0.09 | MS (DCI) : 250 (M+H)⁺ |

e) 1-Brom-4-brommethyl-2-methansulfonyl-benzol
9.9 g 1-Brom-2-methansulfonyl-4-methyl-benzol werden in 100 ml Chlorbenzol aufgenommen, 77 mg Benzoylperoxid sowie 7.1 g N-Bromsuccinimid zugegeben und 1 Stunde unter Rückfluß gekocht. Anschließend wird das Solvens im Vakuum entfernt, der Rückstand in 100 ml CH₂Cl₂ aufgenommen und 2 mal mit 50 ml einer gesättigten wäßrigen Na₂CO₃-Lösung und 1 mal mit 50 ml Wasser gewaschen. Über Na₂SO₄ wird getrocknet und das Solvens im Vakuum entfernt. Der Rückstand wird aus 80 ml HEP/30 ml EE umkristallisiert und man erhält 6.9 g eines blaßgelben Feststoffs, mp 120-124 °C.

| | |
|---|---|
| R_{f}(EE/HEP 1:2) = 0.38 | MS (DCI) : 329 (M+H)⁺ |

f) 3-(4-Brom-3-methanesulfonyl-benzyl)-5-chlor-2-phenyl-3H-imidazol-4-carbaldehyd
1.0 g 5-Chloro-2-phenyl-3H-imidazol-4-carbaldehyd (Chem. Pharm. Bull. 1976, 24(5), 960), 1.6 g 1-Brom-4-brommethyl-2-methansulfonyl-benzol und 691 mg K₂CO₃ werden in 25 ml wasserfreiem DMF 18 Stunden bei RT gerührt. Das Reaktionsgemisch wird auf 300 ml einer halbgesättigten wäßrigen NaHCO₃-Lösung gegossen und 3 mal mit je 150 ml EE extrahiert. Über Na₂SO₄ wird getrocknet und das Solvens im Vakuum entfernt. Chromatographie an Kieselgel mit EE/HEP 1:2 liefert 1.2 g eines farblosen Öls.

| | |
|---|---|
| R_{f}(EE/HEP 1:2) = 0.16 | MS (FAB) : 454 (M+H)⁺ |

g) 4'-(4-Chloro-5-formyl-2-phenyl-imidazol-1-ylmethyl)-3'-methansulfonyl-biphenyl-2-sulfonsäure-tert-butylamid
970 mg 3-(4-Brom-3-methanesulfonyl-benzyl)-5-chlor-2-phenyl-3H-imidazol-4-carbaldehyd, 660 mg N-tert.-Butyl-2-dihydroxyboran-2-yl-benzolsulfonamid (J. Med. Chem. 1997, 40, 547), 24 mg Pd(II)-acetat und 56 mg Triphenylphosphin werden in 13 ml Toluol und 3.5 ml Ethanol aufgenommen und 2.1 ml einer wäßrigen 2 M Na₂CO₃-Lösung zugesetzt. 105 Minuten wird unter Rückfluß gekocht, dann läßt man auf RT abkühlen und das Reaktionsgemisch wird in 200 ml einer halbgesätigten wäßrigen NaHCO₃-Lösung aufgenommen. 3 mal wird mit je 150 ml EE extrahiert, über Na₂SO₄ getrocknet und das Solvens im Vakuum entfernt. Chromatographie an Kieselgel mit EE/HEP 1:2 liefert 660 mg eines farblosen Öls.

| | |
|---|---|
| R_{f}(EE/HEP 1:2) = 0.12 | MS (ES) : 587 (M+H)⁺ |

h) 4'-(4-Chloro-5-formyl-2-phenyl-imidazol-1-ylmethyl)-3'-methansulfonyl-biphenyl-2-sulfonamid
650 mg 4'-(4-Chloro-5-formyl-2-phenyl-imidazol-1-ylmethyl)-3'-methansulfonylbiphenyl-2-sulfonsäure-tert-butylamid werden in 5.6 ml Trifluoressigsäure gelöst und 133 µl Anisol zugespritzt. 8 Stunden wird bei RT gerührt, anschließend die flüchtigen Bestandteile im Vakuum entfernt. 3 mal wird der Rückstand erneut in 20 ml Wasser aufgenommen und das Wasser anschließend im Vakuum entfernt. Schließlich wird der Rückstand noch 2 mal in je 30 ml Toluol suspendiert und erneut die flüchtigen Bestandteile im Vakuum entfernt. Man erhält 570 mg eines blaßgelben Feststoffs, der wegen ungenügender Löslichkeit ohne Reinigung weiter umgesetzt wird.
R_{f}(EE/HEP 2:1)= 0.24
i) 4'-[5-Formyl-4-(2-methoxy-ethoxy)-2-phenyl-imidazol-1-ylmethyl]-3'-methansulfonyl-biphenyl-2-sulfonamid
330 mg 4'-(4-Chloro-5-formyl-2-phenyl-imidazol-1-ylmethyl)-3'-methansulfonylbiphenyl-2-sulfonamid und 250 mg NaOH werden in 12.5 ml Methoxyethanol 2 Stunden bei 90 °C gerührt. Das Reaktionsgemisch wird anschließend auf RT abgekühlt und anschließend auf 100 ml einer halbgesättigten wäßrigen NaHCO₃-Lösung gegossen. 3 mal wird mit je 100 ml EE extrahiert, über Na₂SO₄ getrocknet und das Solvens im Vakuum entfernt. Man erhält 350 mg eines farblosen Öls, das wegen überraschender Instabilität ohne Reinigung sofort weiter eingesetzt werden muß.
R_{f}(EE/HEP 2:1) = 0.18
j) 4'-[5-Formyl-4-(2-methoxy-ethoxy)-2-phenyl-imidazol-1-ylmethyl]-3'-methansulfonylbiphenyl-2-sulfonylcyanamid
340 mg 4'-[5-Formyl-4-(2-methoxy-ethoxy)-2-phenyl-imidazol-1-ylmethyl]-3'-methansulfonyl-biphenyl-2-sulfonamid werden nach der allgemeinen Vorschrift zur Herstellung von Sulfonylcyanamiden aus Sulfonamiden umgesetzt (Reaktionszeit 15 Minuten) und nach Chromatographie an Kieselgel mit EE/MeOH 1:5 erhält man 90 mg weißer Kristalle, mp >270 °C.

| | |
|---|---|
| R_{f}(EE/MeOH 1:5) = 0.27 IR (C≡N): 2178.1 cm⁻¹ | MS (FAB) : 595 (M+H)⁺ |

### Beispiel 2:

3'-Chloro-4'-[5-formyl-4-(2-methoxy-ethoxy)-2-phenyl-imidazol-1-ylmethyl]-biphenyl-2-sulfonylcyanamid 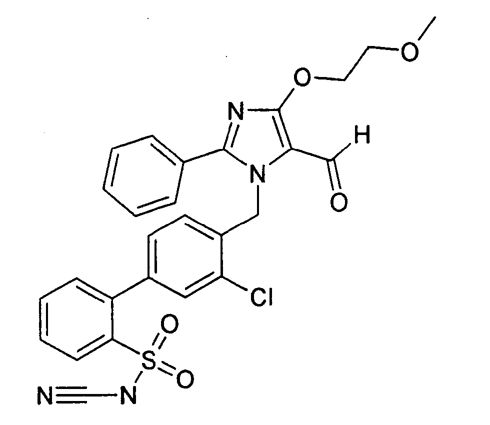
a) 4-Brom-1-brommethyl-2-chlor-benzol
7.1 ml 4-Brom-2-chlortoluol werden in 20 ml Chlorbenzol gelöst und bei 130°C portionsweise mit einer Mischung aus 9.4 g N-Bromsuccinimid und 200 mg Dibenzoylperoxid versetzt. 30 Minuten wird unter Rückfluß gekocht, nach dem Abkühlen mit 100 ml CH₂Cl₂ verdünnt und je 1 mal mit 50 ml einer gesättigten wäßrigen Na₂SO₃-Lösung und 100 ml einer gesättigten wäßrigen NaHCO₃-Lösung gewaschen. Über Na₂SO₄ wird getrocknet und das Solvens im Vakuum entfernt. Man erhält 11.0 g eines blaßgelben Öls.

| | |
|---|---|
| R_{f} (EE/HEP 1:8) = 0.49 | MS (DCI) : 283 (M+H)⁺ |

b) 3-(4-Brom-2-chlor-benzyl)-5-chlor-2-phenyl-3H-imidazol-4-carbaldehyd
1.5 g 4-Chloro-5-formyl-2-phenyl-imidazol (Chem. Pharm. Bull. 1976, 24(5), 960), 5.8 g K₂C0₃ und 8.0 g 4-Brom-1-brommethyl-2-chlor-benzol werden in 50 ml DMF 24 h bei RT gerührt. Anschließend wird mit 250 ml EE verdünnt und 2 mal mit je 100 ml Wasser und 1 mal mit 100 ml einer gesättigten wäßrigen NaCl-Lösung gewaschen. Über Na₂SO₄ wird getrocknet und die Solventien im Vakuum entfernt.
Chromatographie an Kieselgel mit EE/HEP 1:4 liefert 2.2 g eines farblosen Öls.

| | |
|---|---|
| R_{f} (EE/HEP 1:4) = 0.47 | MS (ES) : 411 (M+H)⁺ |

c) 3'-Chloro-4'-(4-chloro-5-formyl-2-phenyl-imidazol-1-ylmethyl)-biphenyl-2-sulfonsäure-tert-butylamid
2.2 g 3-(4-Brom-2-chlor-benzyl)-5-chlor-2-phenyl-3H-imidazol-4-carbaldehyd, 2.0 g N-tert-Butyl-2-dihydroxyboran-2-yl-benzolsulfonamid ( J. Med. Chem. 1997, 40, 547), 140 mg Triphenylphosphin, 64 mg Pd(II)-acetat sowie 1.2 g Na₂CO₃ werden in 30 ml Toluol, 10 ml Ethanol sowie 10 ml Wasser gelöst und 3 h unter Rückfluß gekocht. Nach dem Abkühlen werden 200 ml einer gesättigten wäßrigen NaHCO₃-Lösung zugegeben und 3 mal mit je 200 ml EE extrahiert. Über MgSO₄ wird getrocknet und das Solvens im Vakuum entfernt. Chromatographie an Kieselgel liefert 1.5 g eines farblosen Schaums.

| | |
|---|---|
| R_{f} (DIP) = 0.25 | MS (ES) : 542 (M+H)⁺ |

d) 3'-Chloro-4'-(4-chloro-5-formyl-2-phenyl-imidazol-1-ylmethyl)-biphenyl-2-sulfonamid
1.5 g 3'-Chloro-4'-(4-chloro-5-formyl-2-phenyl-imidazol-1-ylmethyl)-biphenyl-2-sulfonsäure-tert-butylamid und 340 µl Anisol werden in 10 ml Trifluoressigsäure gelöst und bei RT 24 h gerührt. Die flüchtigen Bestandteile werden im Vakuum entfernt, je 2 mal mit 50 ml Toluol aufgenommen und erneut die flüchtigen Bestandteile im Vakuum entfernt. Man erhält 1.5 g eines farblosen Schaums.

| | |
|---|---|
| R_{f} (DIP) = 0.15 | MS (ES) : 486 (M+H)⁺ |

e) 3'-Chloro-4'-[5-formyl-4-(2-methoxy-ethoxy)-2-phenyl-imidazol-1-ylmethyl]-biphenyl-2-sulfonamid
280 mg 3'-Chloro-4'-(4-chlor-5-formyl-2-phenyl-imidazol-1-ylmethyl)-biphenyl-2-sulfonamid und 230 mg NaOH werden in 11.5 ml Methoxyethanol 3 Stunden bei 90 °C gerührt. Nach dem Abkühlen wird das Reaktionsgemisch in 150 ml einer halbgesättigten wäßrigen NaHCO₃-Lösung aufgenommen und 3 mal mit je 100 ml EE extrahiert. Über Na₂SO₄ wird getrocknet und das Solvens im Vakuum entfernt. Man erhält 310 mg eines farblosen Öls, das ohne Reinigung direkt weiter umgesetzt wird.
R_{f}(EE/HEP 2-1) = 0.37
f) 3'-Chloro-4'-[5-formyl-4-(2-methoxy-ethoxy)-2-phenyl-imidazol-1-ylmethyl]-biphenyl-2-sulfonylcyanamid
69 mg 3'-Chloro-4'-[5-formyl-4-(2-methoxy-ethoxy)-2-phenyl-imidazol-1-ylmethyl]-biphenyl-2-sulfonamid werden nach der allgemeinen Vorschrift zur Herstellung von Sulfonylcyanamiden aus Sulfonamiden umgesetzt (Reaktionszeit 1 Stunde) und nach Chromatographie an Kieselgel mit EE/MeOH 1:5 erhält man 63 mg weißer Kristalle, mp 195 °C (unter Zersetzung).

| | | |
|---|---|---|
| R_{f}(EE/MeOH 1:5) = 0.33 | IR (C≡N): 2178.6 cm⁻¹ | MS (ES): 552 (M+H)⁺ |

### Beispiel 3:

4'-[5-Formyl-4-(2-methoxy-ethoxy)-2-phenyl-imidazol-1-ylmethyl]-3'-(2-methoxyethoxy)-biphenyl-2-sulfonylcyanamid 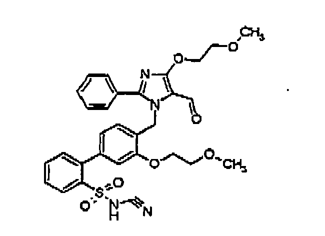
a) 4-Methyl-3-(2-methoxy)ethoxy-anilin
22.0 g 3-Hydroxy-4-methyl-anilin, 24.9 g 2-Bromethylmethylether und 233 g Cs₂CO₃ werden in 570 ml DMF gelöst und 8 h bei 40°C gerührt. 3 l einer 10% wäßrigen Natriumhydrogencarbonat-Lösung werden zugegeben, 6 mal mit je 750 ml EE extrahiert und 2 mal mit je 1 l einer 10% wäßrigen Natriumhydrogencarbonat-Lösung gewaschen. Über Natriumsulfat wird getrocknet und das Solvens im Vakuum entfernt. Man erhälöt 32.9 g eines gelben Öls, das so weiter eingesetzt wird.
R_{f}(EE/HEP 1:1) = 0.33
b) 4-Methyl-3-(2-methoxy)ethoxy-brombenzol
32.8 g 4-Methyl-3-(2-methoxy)ethoxy-anilin werden in 660 ml einer halbgesättigten wäßrigen HBr-Lösung suspendiert und bei 0°C eine Lösung von 12.5 g NaNO₃ in 25 ml Wasser langsam zugetropft. 30 Minuten wird bei 0°C nachgerührt und anschließend diese Lösung zu einer auf 50°C erwärmten Lösung von 51.9 g CuBr in 490 ml einer gesättigten wäßrigen HBr-Lösung langsam zugegeben. Das Reaktionsgemisch wird anschließend über einen Zeitraum von 6 h langsam von 50°C auf 70°C erwärmt. Nach anschließendem Abkühlen wird 4 mal mit je 500 ml Diethylether extrahiert, mit 500 ml einer gesättigten wäßrigen NaCl-Lösung gewaschen und über Natriumsulfat getrocknet. Chromatographie an Kieselgel mit EE/HEP 1:8 liefert 15.4 g eines farblosen Öls.

| | |
|---|---|
| R_{f}(EE/HEP 1:1) = 0.41 | MS (DCl) : 245 (M+H)⁺ |

c) 4-Brommethyl-3-(2-methoxy)ethoxy-bromberizol
15.3 g 4-Methyl-3-(2-methoxy)ethoxy-brombenzol werden in 300 ml Chlorbenzol gelöst und unter Rückfluß ein Gemisch aus 11.1 g N-Bromsuccinimid und 125 mg Benzoylperoxid portionsweise zugegeben. 24 h wird unter Rückfluß gekocht, das Solvens im Vakuum entfernt und anschließend mit 500 ml CH₂Cl₂ aufgenommen. Mit zunächst 200 ml einer gesättigten wäßrigen Na₂SO₄-Lösung, anschließend mit 100 ml einer gesättigten wäßrigen Natriumcarbonat-Lösung wird gewaschen. Über Natriumsulfat wird getrocknet und das Solvens im Vakuum entfernt.
Chromatographie an Kieselgel mit EE/HEP 1:15 liefert 12.8 g eines blaßgelben Öls.

| | |
|---|---|
| R_{f}(EE/HEP 1:4) = 0.42 | MS (DCI) : 323 (M+H)⁺ |

d) 3-[4-Brom-2-(2-methoxy-ethoxy)-benzyl]-5-chlor-2-phenyl-3H-imidazol-4-carbaldehyd
620 mg 5-Chlor-2-phenyl-3H-imidazol-4-carbaldehyd, 970 mg 4-Brom-1-brommethyl-2-(2-methoxy-ethoxy)-benzol und 415 mg K₂CO₃ werden in 15 ml wasserfreiem DMF suspendiert und 15 Stunden bei RT gerührt. Mit 75 ml einer gesättigten wäßrigen Na₂CO₃-Lösung sowie 75 ml Wasser wird aufgenommen und 3 mal mit je 100 ml EE extrahiert. Über Na₂SO₄ wird getrocknet und das Solvens im Vakuum entfernt. Chromatographie an Kieselgel mit EE/HEP 1:4 liefert 640 mg eines farblosen Öls.

| | |
|---|---|
| R_{f}(EE/HEP 1:2) = 0.26 | MS (ES) : 450 (M+H)⁺ |

e) 4'-(4-Chlor-5-formyl-2-phenyl-imidazol-1-ylmethyl)-3'-(2-methoxy-ethoxy)-biphenyl-2- sulfonsäure-tert-butylamid
620 mg 3-[4-Brom-2-(2-methoxy-ethoxy)-benzyl]-5-chlor-2-phenyl-3H-imidazol-4-carbaldehyd, 420 mg 2-Dihydroxyboryl-benzolsulfonsäure-tert-butylamid, 16 mg Pd-(II)-acetat und 36 mg Triphenylphosphin werden in 8 ml Toluol sowie 2 ml Ethanol gelöst und 1.4 ml einer 2N wäßrigen Na₂CO₃-Lösung zugegeben 4 Stunden wird unter Rückfluß gekocht und nach Abkühlung das Reaktionsgemisch mit 75 ml einer gesättigten wäßrigen Na₂CO₃-Lösung sowie 75 ml Wasser verdünnt. 3 mal wird mit je 100 ml EE extrahiert. Über Na₂SO₄ wird getrocknet und das Solvens im Vakuum entfernt. Chromatographie an Kieselgel mit EE/HEP 1:2 liefert 600 mg eines farblosen Öls.

| | |
|---|---|
| R_{f}(EE/HEP 1:2) = 0.20 | MS (ES) : 583 (M+H)⁺ |

f) 4'-(4-Chlor-5-formyl-2-phenyl-imidazol-1-ylmethyl)-3'-(2-methoxy-ethoxy)-biphenyl-2- sulfonamid
570 mg 4'-(4-Chlor-5-formyl-2-phenyl-imidazol-1-ylmethyl)-3'-(2-methoxy-ethoxy)-biphenyl-2- sulfonsäure-tert-butylamid werden in 5 ml Trifluoressigsäure gelöst und 117 µl Anisol zugegeben. 8 h wird bei RT gerührt, anschließend das Solvens im Vakuum entfernt. Chromatographie an Kieselgel mit EE/HEP 1:1 liefert 350 mg eines amorphen Feststoffs.

| | |
|---|---|
| R_{f}(EE/HEP 1:1) = 0.27 | MS (ES) : 527 (M+H)⁺ |

g) 4'-[5-Formyl-4-(2-methoxy-ethoxy)-2-phenyl-imidazol-1-ylmethyl]-3'-(2-methoxyethoxy)-biphenyl-2-sulfonamid
340 mg 4'-(4-Chlor-5-formyl-2-phenyl-imidazol-1-ylmethyl)-3'-(2-methoxy-ethoxy)-biphenyl-2- sulfonamid und 260 mg NaOH werden in 13 ml 2-Methoxyethanol gelöst und 7 Stunden bei 90°C gerührt. Nach Abkühlung wird das Reaktionsgemisch mit 100 ml einer 10% wäßrigen NaHCO₃-Lösung verdünnt und 3 mal mit je 100 ml EE extrahiert. Über Na₂SO₄ wird getrocknet und das Solvens im Vakuum entfernt. Chromatographie an Kieselgel mit EE/HEP 2:1 liefert 230 mg eines amorphen Feststoffs.

| | |
|---|---|
| R_{f}(EE/HEP 2:1)= 0.33 | MS (ES) : 566 (M+H)⁺ |

h) 4'-[5-Formyl-4-(2-methoxy-ethoxy)-2-phenyl-imidazol-1-ylmethyl]-3'-(2-methoxy-ethoxy)-biphenyl-2-sulfonylcyanamid
210 mg 4'-[5-Formyl-4-(2-methoxy-ethoxy)-2-phenyl-imidazol-1-ylmethyl]-3'-(2-methoxy-ethoxy)-biphenyl-2-sulfonamid sowie 154 mg K₂CO₃ werden in 4 ml wasserfreiem Acetonitril suspendiert und 74 µl einer 5N Lösung von BrCN in Acetonitril zugespritzt. 3 Stunden wird unter Rückfluß gekocht und nach Abkühlung das gesamte Reaktionsgemisch an Kieselgel chromatographiert mit EE/MeOH 10:1.
Man erhält 170 mg weißer Kristalle, mp 162°C.

| | |
|---|---|
| R_{f}(EE/MeOH 10:1) = 0.11 | MS (ES): 591 (M+H)⁺ |

### Pharmakologische Daten:

### Inhibition des Na⁺- abhängigen Cl⁻/HCO3⁻ - Austauschers (NCBE) in humanen Endothelzellen

Humane Endothelzellen (ECV-304) wurden aus Kulturflaschen mit Hilfe von Trypsin/EDTA-Puffer (0,05/0,02% in Phoshatpuffer) abgelöst und nach Zentrifugation (100g, 5 min) in einer gepufferten Salzlösung (mmol/l: 115 NaCl, 20 NH₄Cl, 5 KCl, 1 CaCl₂, 1 MgSO₄, 20 N-(2-Hydroxyethyl)piperazin-N=-2-ethansulfonsäure (HEPES), 5 Glucose und 1 g/l Rinderserumalbumin; pH 7,4) aufgenommen. Diese Zellsuspension wurde mit 5 µM BCECF-acetoxymethylester für 20 min bei 37°C inkubiert. Anschließend wurden die Zellen gewaschen und in einer Natrium- und Bicarbonatfreien Pufferlösung (mmol/l: 5 HEPES, 133,8 Cholinchlorid, 4,7 KCI, 1,25 MgCl₂, 0,97 K₂HPO₄, 0,23 KH₂PO₄, 5 Glucose; pH 7,4) resuspendiert.
Für die nachfolgende Fluoreszenzmessung im FLIPR (Fluorescent Imaging Plate Reader) wurden pro well einer 96-well-Mikrotiterplatte 100 µl dieser Zellsuspension mit jeweils 20000 Zellen einpipettiert und diese Mikrotiterplatte zentrifugiert (100g, 5 min).
Im FLIPR wurden nun aus einer weiteren vorbereiteten Mikrotiterplatte jeweils 100 µl Pufferlösung entnommen und in jedes der 96 wells der Meßplatte einpipettiert. Dabei wurde für eine 100% Kontrolle, d.h. eine Erholung des intrazellulären pH (pHᵢ) über den NCBE, eine Bicarbonat-und Natrium-haltige Pufferlösung (mmol/l: 5 HEPES, 93,8 NaCl, 40 NaHCO₃, 4,7 KCl, 1,25 CaCl₂, 1,25 MgCl₂, 0,97 Na₂HPO₄, 0,23 NaH₂PO₄, 5 Glucose; pH 7,4) verwendet, die 50 µM HOE 642 enthielt. Für eine 0% Kontrolle, d.h. keinerlei pHᵢ-Erholung, wurde eine Bicarbonat-freie, Natrium-haltige Pufferlösung (mmol/l: 5 HEPES, 133,8 NaCl, 4,7 KCl, 1,25 CaCl₂, 1,25 MgCl₂, 0,97 Na₂HPO₄, 0,23 NaH₂PO₄, 5 Glucose; pH 7,4) eingesetzt, welcher ebenfalls 50 µM HOE 642 zugefügt waren. Die erfindungsgemäßen Verbindungen wurden in verschiedenen Konzentrationen der Natrium-und Bicarbonat-haltigen Lösung zugesetzt.
Nach Zugabe der Pufferlösungen zu den in der Meßplatte befindlichen farbstoffbeladenen, angesäuerten Zellen wurde der Anstieg der Fluoreszenzintensität, welcher einem Anstieg des pHᵢ entsprach, in jedem well der Mikrotiterplatte bestimmt. Die Kinetiken wurden dabei über einen Zeitraum von 2 Minuten bei 35°C aufgenommen.
Die Zunahme der Fluoreszenzintensitäten für unterschiedliche Konzentrationen der erfindungsgemäßen Verbindungen wurde auf die beiden Kontrollen bezogen und daraus die Hemmwirkung der Substanzen ermittelt.

### Ergebnisse

### Restaktivität des NCBE bei einer Inhibitorkonzentration von 10 µM (in %)

Verbindung des Beispiels Nr.

| | |
|---|---|
| 1 | 9,8 |
| 2 | 18,9 |
| 3 | 13,5 |

## Patentansprüche

1. Verbindungen der Formel I, 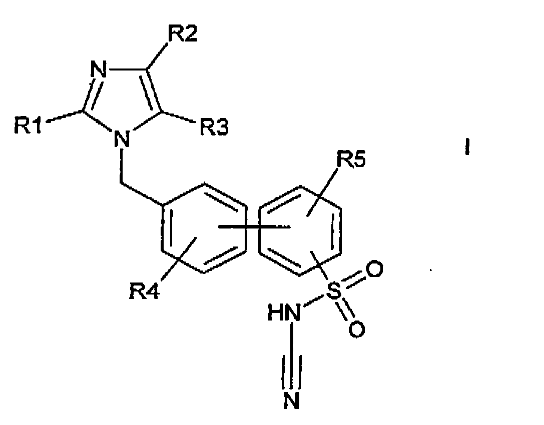 in welcher die Symbole folgende Bedeutung haben:
R(1) Alkyl mit 1, 2, 3, 4, 5, 6, 7 oder 8 C-Atomen, -C_{d}H_{2d}-Cycloalkyl mit 3, 4, 5, 6 oder 7 C-Atomen mit d gleich Null, 1 oder 2 oder -CₐH₂ₐ-Phenyl,
wobei der Phenylteil unsubstituiert oder substituiert ist mit 1, 2 oder 3 gleichen oder verschiedenen Resten aus der Reihe F, Cl, Br, I, CF₃, Methyl, Methoxy, Hydroxy oder NR(8)R(9);
R(8) und R(9) unabhängig voneinander Wasserstoff oder Alkyl mit 1, 2, 3 oder 4 C-Atomen;
a Null, 1 oder 2;
R(2) O-(Alkylen mit 2, 3, oder 4 C-Atomen)-O-R(17) oder NR(50)R(51);
R(17) Wasserstoff oder Alkyl mit 1, 2, 3, 4, 5, 6, 7 oder 8 C-Atomen;
R(50) und R(51) unabhängig voneinander
-(Alkylen mit 2, 3, oder 4 C-Atomen)-O-R(52);
R(52) Wasserstoff oder Alkyl mit 1, 2, 3, 4, 5, 6, 7 oder 8 C-Atomen;
R(3) Wasserstoff, -CN, oder CO-R(6);
R(6) Wasserstoff, Alkyl mit 1, 2, 3, 4, 5, 6, 7 oder 8 C-Atomen oder OR(30);
R(30) Wasserstoff oder Alkyl mit 1, 2, 3, 4, 5, 6, 7 oder 8 C-Atomen;
R(4) Wasserstoff, Cl, F, Br, I, SOₚ-R(16) oder O-CH₂-CH₂-O-R(33);
p Null, 1 oder 2;
R(16) Alkyl mit 1, 2, 3, 4, 5, 6, 7 oder 8 C-Atomen oder Phenyl, das unsubstituiert oder substituiert ist mit 1, 2 oder 3 gleichen oder verschiedenen Resten aus der Reihe F, Cl, Br, I, CF₃, Methyl, Methoxy, Hydroxy oder NR(26)R(27);
R(26) und R(27) unabhängig voneinander Wasserstoff oder Alkyl mit 1, 2, 3 oder 4 C-Atomen;
R(33) Methyl oder Ethyl;
R(5) Wasserstoff;
sowie deren physiologisch verträglichen Salze.

2. Verbindungen der Formel I gemäß Anspruch 1, in denen bedeutet:
R(1) -CₐH₂ₐ-Phenyl, wobei der Phenylteil unsubstituiert oder substituiert ist mit 1 oder 2 gleichen oder verschiedenen Resten aus der Reihe F, Cl, Br, CF₃, Methyl, Methoxy, Hydroxy oder NR(8)R(9);
R(8) und R(9) unabhängig voneinander Wasserstoff oder Methyl;
a Null oder 1;
R(2) O-(Alkylen mit 2, 3, oder 4 C-Atomen)-O-R(17) oder NR(50)R(51);
R(17) Alkyl mit 1, 2, 3 oder 4 C-Atomen;
R(50) und R(51) unabhängig voneinander -(Alkylen mit 2, 3, oder 4 C-Atomen)-O-R(52);
R(52) Alkyl mit 1, 2, 3, oder 4 C-Atomen;
R(3) CO-R(6);
R(6) Wasserstoff;
R(4) SO₂R(16) mit R(16) gleich Alkyl mit 1, 2, 3 oder 4 C-Atomen oder O-CH₂-CH₂ O-CH₃;
R(5) Wasserstoff;
sowie deren physiologisch verträglichen Salze.

3. Verbindungen der Formel I gemäß einem oder mehreren der Ansprüche 1 bis 2, in denen R(2) O-CH₂-CH₂-O-R(17) oder NR(50)R(51) bedeutet mit R(97) gleich Alkyl mit 1, 2, 3 oder 4 C-Atomen und R(50) und R(51) gleich -CH₂-CH₂-O-R(52) mit R(52) gleich Alkyl mit 1, 2, 3 oder 4 C-Atomen, sowie deren physiologisch verträglichen Salze.

4. Verbindungen der Formel I gemäß einem oder mehreren der Ansprüche 1 bis 3 und/oder ihre physiologisch verträglichen Salze zur Anwendung als Arzneimittel.

5. Pharmazeutische Zubereitung, **gekennzeichnet durch** einen wirksamen Gehalt einer Verbindung der Formel I nach einem oder mehreren der Ansprüche 1 bis 3 und/oder eines physiologisch verträglichen Salzes davon.

6. Pharmazeutische Zubereitung gemäß Anspruch 5, **dadurch gekennzeichnet, daß** sie zusätzlich eine wirksame Menge eines NHE-Inhibitors und/oder eine wirksame Substanz aus einer anderen Herz-Kreislauf-Wirkstoffklasse, und/oder deren physiologisch verträglichen Salze enthält.

7. Verbindungen der Formel I gemäß einem oder mehreren der Ansprüche 1 bis 3 und/oder ihre physiologisch verträglichen Salze zur Verwendung als Inhibitor des Natrium-abhängigen Bicarbonat/Chlorid-Austauschers.

8. Verbindungen der Formel I gemäß einem oder mehreren der Ansprüche 1 bis 3 und/oder ihre physiologisch verträglichen Salze zur Verwendung in der Theraphie und/oder Prophylaxe des Herzinfarkts, der Angina Pectoris, von durch ischämische Zustände hervorgerufenen Krankheiten, von gestörtem Atemantrieb, von ischämischen Zuständen des Herzens, von ischämischen Zuständen des peripheren und zentralen Nervensystems und des Schlaganfalls, von ischämischen Zuständen peripherer Organe und Gliedmaßen, von Krankheiten, bei denen die Zellproliferation eine primäre oder sekundäre Ursache darstellt oder in der Behandlung von Schockzuständen, oder zum Einsatz bei chirurgischen Operationen und Organtransplantationen oder zur Konservierung und Lagerung von Transplantaten für chirurgische Maßnahmen.

9. Verbindungen der Formel I gemäß einen oder mehreren der Ansprüche 1 bis 3 und/oder ihre physiologisch verträglichen Salze zur Verwendung als Antiatherosklerotikum, Mittel gegen diabetische Spätkomplikationen, Krebserkrankungen, fibrotische Erkrankungen oder Organhypertrophien und/oderhyperplasien.

10. Pharmazeutische Zubereitung gemäß Anspruch 5 und/oder 6 zur Verwendung in der Theraphie und/oder Prophylaxe des Herzinfarkts, der Angina Pectoris, von durch ischämische Zustände hervorgerufenen Krankheiten, von gestörtem Atemantrieb, von ischämischen Zuständen des Herzens, von ischämischen Zuständen des peripheren und zentralen Nervensystems und des Schlaganfalls, von ischämischen Zuständen peripherer Organe und Gliedmaßen, von Krankheiten, bei denen die Zellproliferation eine primäre oder sekundäre Ursache darstellt oder in der Behandlung von Schockzuständen, oder zum Einsatz bei chirurgischen Operationen und Organtransplantationen oder zur Konservierung und Lagerung von Transplantaten für chirurgische Maßnahmen.

11. Phamazeutische Zubereitung gemäß Anspruch 5 und/oder 6 zur Verwendung als Antiatherosklerotikum, Mittel gegen diabetische Spätkomplikationen, Krebserkrankungen, fibrotische Erkrankungen oder Organhypertrophien und/oderhyperplasien,

## Claims

1. A compound of the formula I 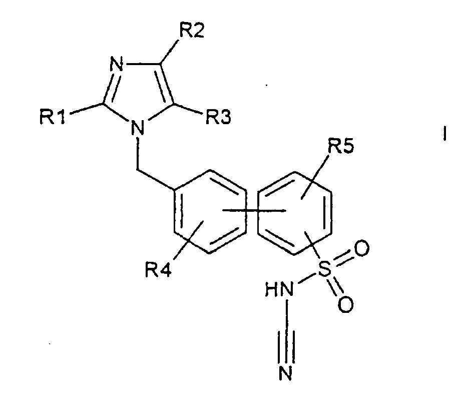 in which the symbols have the following meaning:
R(1) is alkyl having 1, 2, 3, 4, 5, 6, 7 or 8 carbon atoms, -C_{d}H_{2d}-cycloalkyl having 3, 4, 5, 6 or 7 carbon atoms where d is equal to zero, 1 or 2 or -CₐH₂ₐ-phenyl,
where the phenyl moiety is unsubstituted or substituted by 1, 2 or 3 identical or different radicals from the group consisting of F, Cl, Br, I, CF₃, methyl, methoxy, hydroxyl or NR(8)R(9);
R(8) and R(9) independently of one another are hydrogen or alkyl having 1, 2, 3 or 4 carbon atoms;
a is zero, 1 or 2;
R(2) is O-(alkylene having 2, 3 or 4 carbon atoms)-O-R(17) or NR(50)R(51);
R(17) is hydrogen or alkyl having 1, 2, 3, 4, 5, 6, 7 or 8 carbon atoms;
R(50) and R(51) independently of one another are -(alkylene having 2, 3 or 4 carbon atoms)-O-R(52);
R(52) is hydrogen or alkyl having 1, 2, 3, 4, 5, 6, 7 or 8 carbon atoms;
R(3) is hydrogen, -CN or CO-R(6);
R(6) is hydrogen, alkyl having 1, 2, 3, 4, 5, 6, 7 or 8 carbon atoms or OR(30);
R(30) is hydrogen or alkyl having 1, 2, 3, 4, 5, 6, 7 or 8 carbon atoms;
R(4) is hydrogen, Cl, F, Br, I, SOₚ-R(16) or O-CH₂-CH₂-O-R(33);
p is zero, 1 or 2;
R(16) is alkyl having 1, 2, 3, 4, 5, 6, 7 or 8 carbon atoms or phenyl which is unsubstituted or substituted by 1, 2 or 3 identical or different radicals from the group consisting of F, Cl, Br, I, CF₃, methyl, methoxy, hydroxyl or NR(26)R(27);
R(26) and R(27) independently of one another are hydrogen or alkyl having 1, 2, 3 or 4 carbon atoms;
R(33) is methyl or ethyl;
R(5) is hydrogen;
or its physiologically tolerable salts.

2. A compound of the formula I as claimed in claim 1, in which:
R(1) is -CₐH₂ₐ-phenyl, where the phenyl moiety is unsubstituted or substituted by 1 or 2 identical or different radicals from the group consisting of F, Cl, Br, CF₃, methyl, methoxy, hydroxyl or NR(8)R(9); R(8) and R(9) independently of one another are hydrogen or methyl;
a is zero or 1;
R(2) is O-(alkylene having 2, 3 or 4 carbon atoms)-O-R(17) or
NR(50)R(51);
R(17) is alkyl having 1, 2, 3 or 4 carbon atoms;
R(50) and R(51) independently of one another are -(alkylene having 2, 3 or 4 carbon atoms)-O-R(52);
R(52) is alkyl having 1, 2, 3 or 4 carbon atoms;
R(3) is CO-R(6);
R(6) is hydrogen;
R(4) is SO₂R(16) where R(16) is alkyl having 1, 2, 3 or 4 carbon atoms, or O-CH₂-CH₂-O-CH₃;
R(5) is hydrogen;
or its physiologically tolerable salts.

3. A compound of the formula I as claimed in one or more of claims 1 to 2, in which R(2) is O-CH₂-CH₂-O-R(17) or NR(50)R(51) where R(17) is equal to alkyl having 1, 2, 3 or 4 carbon atoms and R(50) and R(51) are equal to -CH₂-CH₂-O-R(52) where R(52) is equal to alkyl having 1, 2, 3 or 4 carbon atoms, or its physiologically tolerable salts.

4. A compound of the formula I as claimed in one or more of claims 1 to 3 and/or its physiologically tolerable salts for use as a pharmaceutical.

5. A pharmaceutical preparation which comprises an efficacious amount of a compound of the formula I as claimed in one or more of claims 1 to 3 and/or of a physiologically tolerable salt thereof.

6. The pharmaceutical preparation as claimed in claim 5, which additionally contains an efficacious amount of an NHE inhibitor and/or an active substance from another class of cardiovascular active compound, and/or its physiologically tolerable salts.

7. A compound of the formula I as claimed in one or more of claims 1 to 3 and/or its physiologically tolerable salts for use as an inhibitor of the sodium-dependent bicarbonate/chloride exchanger.

8. A compound of the formula I as claimed in one or more of claims 1 to 3 and/or its physiologically tolerable salts for use in the therapy and/or prophylaxis of cardiac infarct, of angina pectoris, of illnesses caused by ischemic conditions, of disturbed respiratory drive, of ischemic conditions of the heart, of ischemic conditions of the peripheral and central nervous system and of stroke, of ischemic conditions of peripheral organs and limbs, of illnesses in which cell proliferation is a primary or secondary cause or in the treatment of states of shock, or for use in surgical operations and organ transplantations or for the preservation and storage of transplants for surgical measures.

9. A compound of the formula I as claimed in one or more of claims 1 to 3, and/or its physiologically tolerable salts for use as an antiatherosclerotic, agent against diabetic late complications, carcinomatous disorders, fibrotic disorders or organ hypertrophy and/or hyperplasia.

10. A pharmaceutical preparation as claimed in claim 5 and/or 6 for use in the therapy and/or prophylaxis of cardiac infarct, of angina pectoris, of illnesses caused by ischemic conditions, of disturbed respiratory drive, of ischemic conditions of the heart, of ischemic conditions of the peripheral and central nervous system and of stroke, of ischemic conditions of peripheral organs and limbs, of illnesses in which cell proliferation is a primary or secondary cause or in the treatment of states of shock, or for use in surgical operations and organ transplantations or for the preservation and storage of transplants for surgical measures.

11. A pharmaceutical preparation as claimed in claim 5 and/or 6 for use as antiatherosclerotic, agent against diabetic late complications, carcinomatous disorders, fibrotic disorders or organ hypertrophy and/or hyperplasia.

## Revendications

1. Composés de formule I 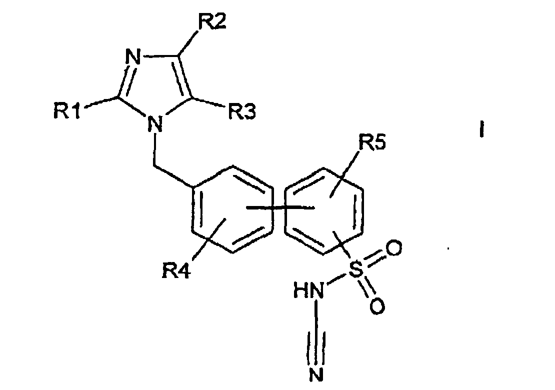 dans laquelle les symboles présentent la signification suivante :
R(1) représente alkyle comprenant 1, 2, 3, 4, 5, 6, 7 ou 8 atomes de carbone, -C_{d}H_{2d}-cycloalkyle comprenant 3, 4, 5, 6 ou 7 atomes de carbone avec d valant zéro, 1 ou 2 ou -CₐH₂ₐ-phényle,
où la partie phényle est non substituée ou substituée par 1, 2 ou 3 radicaux identiques
ou différents de la série F, Cl, Br, I, CF₃, méthyle, méthoxy, hydroxy ou NR(8)R(9) ;
R(8) et R(9) représentent, indépendamment l'un de l'autre,
hydrogène ou alkyle comprenant 1, 2, 3 ou 4 atomes de carbone ;
a vaut zéro, 1 ou 2;
R(2) représente O-(alkylène comprenant 2, 3, ou 4 atomes de carbone)-O-R(17) ou NR(50)R(51) ;
R(17) représente hydrogène ou alkyle comprenant 1, 2, 3, 4, 5, 6, 7 ou 8 atomes de carbone ;
R(50) et R(51) représentent indépendamment l'un de l'autre
-(alkylène comprenant 2, 3, ou 4 atomes de carbone)-O-R(52) ;
R(52) représente hydrogène ou alkyle comprenant 1, 2, 3, 4, 5, 6, 7 ou 8 atomes de carbone ;
R(3) représente hydrogène, -CN, ou CO-R(6) ;
R(6) représente hydrogène, alkyle comprenant 1, 2, 3, 4, 5, 6, 7 ou 8 atomes de carbone ou OR(30) ;
R(30) représente hydrogène ou alkyle comprenant 1, 2, 3, 4, 5, 6, 7 ou 8 atomes de carbone ;
R(4) représente Cl, F, Br, I, SOₚ-R(16) ou O-CH₂-CH₂-O-R(33) ;
p vaut zéro, 1 ou 2 ;
R(16) représente alkyle comprenant 1, 2, 3, 4, 5, 6, 7 ou 8 atomes de carbone ou phényle, qui est non substitué ou substitué par 1, 2 ou 3 radicaux identiques ou différents de la série F, Cl, Br, I, CF₃, méthyle, méthoxy, hydroxy ou NR(26)R(27) ;
R(26) et R(27) représentent, indépendamment l'un de l'autre, hydrogène ou alkyle comprenant 1, 2, 3 ou 4 atomes de carbone ;
R(33) représente méthyle ou éthyle ;
R(5) représente hydrogène ;
ainsi que leurs sels physiologiquement acceptables.

2. Composés de formule I selon la revendication 1, dans laquelle :
R(1) représente -CₐH₂ₐ-phényle, où la partie phényle est non substituée ou substituée par 1 ou 2 radicaux identiques ou différents de la série F, Cl, Br, CF₃, méthyle, méthoxy, hydroxy ou NR(8)R(9) ;
R(8) et R(9) représentent, indépendamment l'un de l'autre, hydrogène ou méthyle ;
a vaut zéro ou 1 ;
R(2) représente O-(alkylène comprenant 2, 3, ou 4 atomes de carbone)-O-R(17) ou NR(50)R(51) ;
R(17) représente alkyle comprenant 1, 2, 3 ou 4 atomes de carbone ;
R(50) et R(51) représentent indépendamment l'un de l'autre
-(alkylène comprenant 2, 3, ou 4 atomes de carbone)-O-R(52) ;
R(52) représente alkyle comprenant 1, 2, 3 ou 4 atomes de carbone ;
R(3) représente CO-R(6) ;
R(6) représente hydrogène ;
R(4) représente SO₂R(16) avec R(16) représentant alkyle comprenant 1, 2, 3 ou 4 atomes de carbone ou O-CH₂-CH₂-O-CH₃ ;
R(5) représente hydrogène ;
ainsi que leurs sels physiologiquement acceptables.

3. Composés de formule I selon l'une ou plusieurs des revendications 1 à 2, où R(2) signifie O-CH₂-CH₂-O-R(17) ou NR(50)R(51) avec R(17) représentant alkyle comprenant 1, 2, 3 ou 4 atomes de carbone et R(50) et R(51) représentant -CH₂-CH₂-O-R (52) avec R(52) représentant alkyle comprenant 1, 2, 3 ou 4 atomes de carbone, ainsi que leurs sels physiologiquement acceptablese.

4. Composés de formule I selon l'une ou plusieurs des revendications 1 à 3 et/ou leurs sels physiologiquement acceptables pour une utilisation comme médicament.

5. Préparation pharmaceutique, **caractérisée par** une teneur active en un composé de formule I selon l'une ou plusieurs des revendications 1 à 3 et/ou un sel physiologiquement acceptable de celui-ci.

6. Préparation pharmaceutique selon la revendication 5, **caractérisée en ce qu'**elle contient en outre une quantité active d' un inhibiteur de NHE et/ou une substance active d'une autre classe de substances actives sur la circulation cardiaque, et/ou leurs sels physiologiquement acceptables.

7. Composés de formule I selon l'une ou plusieurs des revendications 1 à 3 et/ou leurs sels physiologiquement acceptables pour une utilisation comme inhibiteur de l'échangeur bicarbonate/chlorure dépendant du sodium.

8. Composés de formule I selon l'une ou plusieurs des revendications 1 à 3 et/ou leurs sels physiologiquement acceptables destinés à une utilisation dans la thérapie et/ou la prophylaxie de l'infarctus cardiaque, de l'angine de poitrine, de maladies provoquées par des états ischémiques, des troubles respiratoires, d'états ischémiques du coeur, d'états ischémiques du système nerveux périphérique et central, de l'attaque, d'états ischémiques d'organes périphériques et de membres, de maladies dans lesquelles la prolifération cellulaire représente une cause primaire ou secondaire ou dans le traitement d'états de choc ou à l'utilisation dans des opérations chirurgicales et des transplantations d'organes ou à la conservation et à l'entreposage de transplants pour des interventions chirurgicales.

9. Composés de formule I selon l'une ou plusieurs des revendications 1 à 3 et/ou leurs sels physiologiquement acceptables pour une utilisation comme agent anti-athéroscléreux, agent contre les complications tardives du diabète, contre les maladies cancéreuses, contre les maladies fibreuses ou contre les hypertrophies et/ou les hyperplasies des organes.

10. Préparation pharmaceutique selon la revendication 5 et/ou 6 destinée à une utilisation dans la thérapie et/ou la prophylaxie de l'infarctus cardiaque, de l'angine de poitrine, de maladies provoquées par des états ischémiques, des troubles respiratoires, d'états ischémiques du coeur, d'états ischémiques du système nerveux périphérique et central, de l'attaque, d'états ischémiques d'organes périphériques et de membres, de maladies dans lesquelles la prolifération cellulaire représente une cause primaire ou secondaire ou dans le traitement d'états de choc ou à l'utilisation dans des opérations chirurgicales et des transplantations d'organes ou à la conservation et à l'entreposage de transplants pour des interventions chirurgicales.

11. Préparation pharmaceutique selon la revendication 5 et/ou 6 destinée à une utilisation comme agent anti-athéroscléreux, agent contre les complications tardives du diabète, contre les maladies cancéreuses, contre les maladies fibreuses ou contre les hypertrophies et/ou les hyperplasies des organes.
